(19) Europäisches Patentamt
European Patent Office
Office européen des brevets

(11) Veröffentlichungsnummer: **0 260 596 A2**

(12) # EUROPÄISCHE PATENTANMELDUNG

(21) Anmeldenummer: **87113220.5**

(51) Int. Cl.⁴: **C07D 473/18** , A61K 31/52

(22) Anmeldetag: **10.09.87**

(30) Priorität: **17.09.86 DE 3631562**

(43) Veröffentlichungstag der Anmeldung: **23.03.88 Patentblatt 88/12**

(84) Benannte Vertragsstaaten: **AT BE CH DE FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Winkelmann, Erhardt, Dr.**
**Brunhildenweg 5**
**D-6233 Kelkheim Taunus(DE)**
Erfinder: **Winkler, Irvin, Dr.**
**In den Eichen 40**
**D-6237 Liederbach(DE)**

(54) **In 9-Stellung substituierte 2-Amino-6-alkoxy-purine, ihre Verwendung, diese Purine enthaltende Arzneimittel und Verfahren zur Herstellung der Purine.**

(57) Verbindungen der Formel

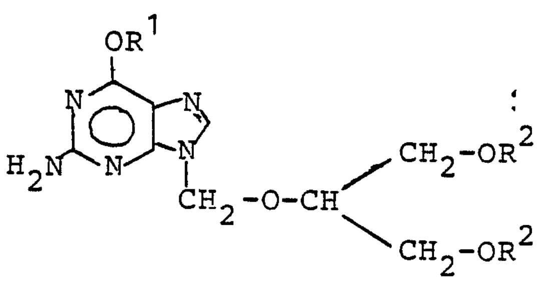

worin
$R^1$ eine Alkylgruppe mit 1 bis 6 Kohlenstoffatomen,
$R^2$ Wasserstoff, eine Benzylgruppe oder eine Acylgruppe CO-$R^3$ ist, wobei
$R^3$ Alkyl mit 1 bis 6 C-Atomen, Benzyl oder Phenyl ist, verschiedene Verfahren zu ihrer Herstellung und diese Verbindungen enthaltende Arzneimittel.
Die Verbindungen eignen sich insbesondere zur Behandlung von Viruserkrankungen.

EP 0 260 596 A2

# In 9-Stellung substituierte 2-Amino-6-alkoxy-purine, ihre Verwendung, diese Purine enthaltende Arzneimittel und Verfahren zur Herstellung der Purine

9-(1,3-Dihydroxy-2-propoxymethyl)-guanin (DHPG, Biolf-62, BW 759U) ist als gutes Antiherpes-Virusmittel bekannt.

Die Erfindung betrifft Verbindungen der Formel 1

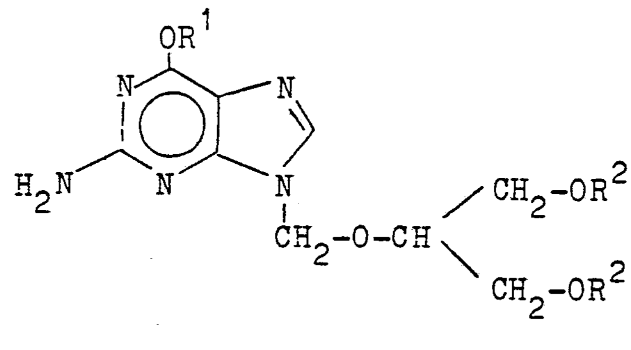

1

und Arzneimittel, die sie als Wirkstoff enthalten, in denen $R^1$ eine Alkylgruppe mit 1 bis 6 Kohlenstoffatomen und $R^2$ Wasserstoff oder eine Benzylgruppe oder ein Acylgruppe $CO\text{-}R^3$ ist, wobei $R^3$ Alkyl mit 1 bis 6 Kohlenstoffatomen, Benzyl oder Phenyl ist.

Gegenüber dem bekannten Guanin-Derivat DHPG, stellen die erfindungsgemäßen Verbindungen durch ihre lipophile 6-Alkoxy-Substitution eine neue Prodrug-Form mit veränderter Pharmakokinetik dar, die bei besonderen Anwendungsformen gegen Herpes-Viren von Vorteil sein können.

Als bevorzugte Verbindung gemäß dem Anspruch sei 2-Amino-6-isopropoxy-9-(1,3-dihydroxy-2-propoxymethyl)-purin genannt.

Die erfindungsgemäßen Verbindungen der Formel 1 können nach verschiedenen Methoden hergestellt werden. Die als Ausgangsstoffe verwendeten 6-Alkoxy-purine, sowie die als Reaktionspartner verwendeten Glycerin-Derivate sind bekannt.

Verfahren A)

Wenn $R^1$ eine Alkylgruppe ist, setzt man zweckmäßig 2-Trimethylsilylamino-6-alkoxy-9-trimethylsilylpurine der Formel 2

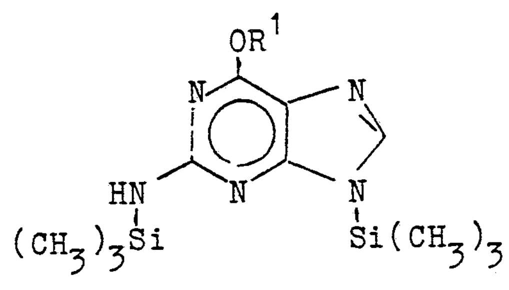

2

mit Halogen-, insbesondere Chlorverbindungen der Formel 3 um,

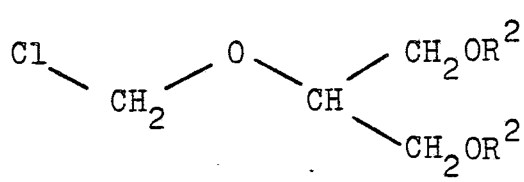

3

wobei $R^2$ Benzyl oder Acyl $COR^3$ und $R^3$ die angegebene Bedeutung hat und man die Trimethylsilyl-bzw. Benzyl-Schutzgruppe abspaltet und den Acylrest gegebenenfalls verseift.

Verfahren B)

Wenn $R^1$ eine Alkylgruppe ist, setzt man zweckmäßig 2-Acetamino-6-alkoxy-9-acetyl-purine der Formel 4

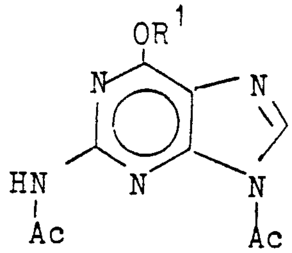

4

mit reaktiven Acetoxy-Verbindungen der Formel 5 um,

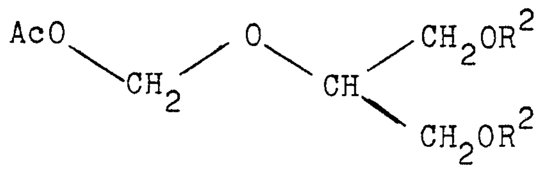

5

wobei $R^2$ Benzyl oder Acyl ist und man die Benzyl-Schutzgruppen abspaltet und die Acylreste verseift.

Verfahren C)

Wenn man 2-Amino-6-chlorpurin der Formel 6, gegebenenfalls als Bis-(trimethylsilyl)-Verbindung der Formel 7

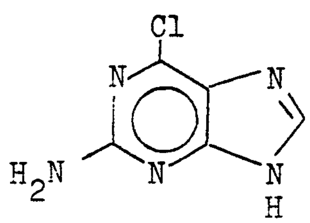

6

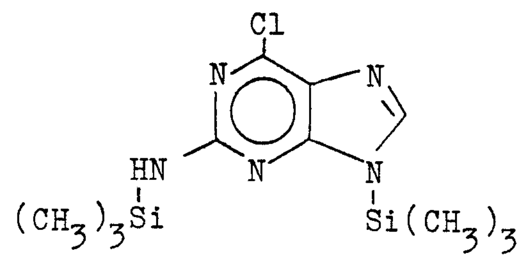

7

mit Halogen-Verbindungen der Formel 3 umsetzt, und die Umsetzungsprodukte der Formel 8 oder 9

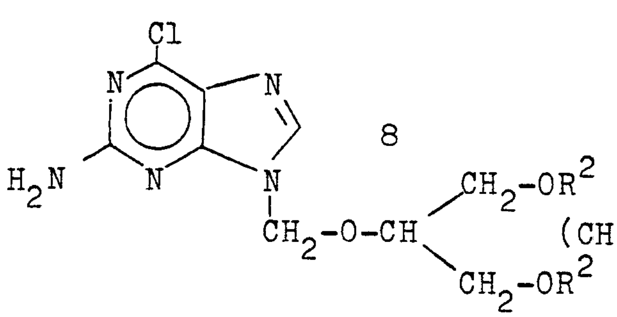

8

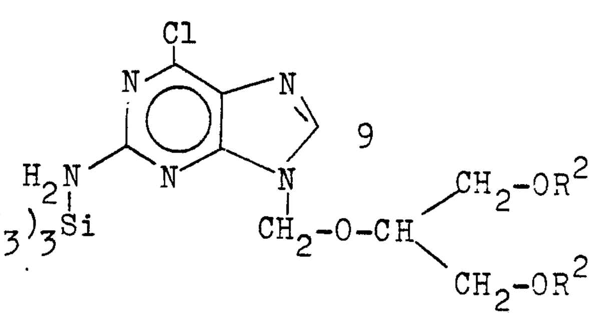

9

nach Abspaltung der Trimethylsilyl-Schutzgruppe in Verbindung 9 mit Alkali-Alkoholaten der Formel 10

Alkalimetall-$OR^1$ 10

wobei $R^1$ eine Alkylgruppe ist, veräthert und die Benzylschutzgruppen $R^2$ abspaltet. Alkalimetall bedeutet dabei Li, Na oder K, vorzugsweise Na oder K.

Die Umsetzungen nach den Verfahren A bis C, die Analogieverfahren darstellen, werden unter den üblichen Bedingungen durchgeführt. Bei den Verfahren A) bis C) liegen die Reaktionstemperaturen im allgemeinen zwischen ca. 0 und 150°C, vorzugsweise zwischen ca. 50 und 100°C. Gewöhnlich arbeitet man bei Normaldruck, jedoch ist es auch möglich, bei erhöhtem oder vermindertem Druck zu arbeiten, wenngleich hiermit normalerweise keine Vorteile verbunden sind. Die Lösungs-und Verteilungsmittel können natürlich sowohl in Form einheitlicher Stoffe als auch in Form von Gemischen verwendet werden. Zu den einzelnen Verfahren wird noch folgendes ausgeführt:

Die Umsetzungen nach Verfahren A) werden zweckmäßig einem Lösungs-oder Verteilungsmittel durchgeführt. Dafür kommen insbesondere aprotische Lösungsmittel in Frage, wie Benzol, Toluol, die verschiedenen Xylole, ferner 1,2-Dichlorethan, Chlorbenzol, 1,2-Dimethoxyethan, Dioxan. Zum Abfangen der gebildeten Halogenwasserstoffsäure empfiehlt sich die Verwendung einer Base, wie Triethylamin oder N-Ethylmorpholin. Die Reaktionszeiten betragen je nach Reaktionstemperatur und Reaktionspartner einige Minuten bis mehrere Stunden, meistens 12 bis 24 Stunden.

Wegen der Feuchtigkeitsempfindlichkeit der als Ausgangsstoffe verwendeten trialkylsilyl-geschützten Purine 2 empfiehlt sich die Verwendung eines Schutzgases im Reaktionsgefäß, z.B. Stickstoff oder Argon. Die Abspaltung der Trialkylsilyl-Schutzgruppen kann z.B. durch Kochen der Zwischenverbindung mit einem Alkohol, wie Methanol oder Ethanol erfolgen.

Die Umsetzungen nach Verfahren B) werden zweckmäßig ebenfalls in einem Lösungs-oder Verteilungsmittel durchgeführt. Dafür kommen insbesondere Sulfone als aprotische Lösungsmittel in Frage, wie Sulfolan (Tetramethylensulfon) oder Dipropylsulfon. Bei dieser Umsetzung empfiehlt sich die Verwendung eines sauren Katalysators, wie Methan-, Ethan-, Benzol-oder Toluol-sulfonsäure. Die Reaktionszeiten betragen je nach Reaktionstemperatur und Reaktionspartner meist mehrere Stunden bis zu einigen Tagen.

Die Umsetzungen nach Verfahren C) werden zweckmäßig in einem Lösungs-oder Verteilungsmittel durchgeführt. Dafür kommen insbesondere aprotische Lösungsmittel in Frage, wie Benzol, Toluol, die verschiedenen Xylole, ferner 1,2-Dichlorethan, Chlorbenzol, 1,2-Dimethoxyethan, Dioxan. Zum Abfangen der gebildeten Halogenwasserstoffsäure empfiehlt sich die Verwendung einer Base, wie Triethylamin oder N-Ethylmorpholin. Die Reaktionszeiten betragen je nach Reaktionstemperatur und Reaktionspartner einige Minuten bis mehrere Stunden, meistens 12 bis 24 Stunden. Bei der Umsetzung nach Verfahren C kann ein Zusatz von katalytischen Mengen von Quecksilber-II-Verbindungen, wie $Hg(CH)_2$ oder Alkalijodiden, wie NaJ von Vorteil sein.

Wegen der Feuchtigkeitsempfindlichkeit der als Ausgangsstoffe verwendeten trialkylsilyl-geschützten Purine 7 empfiehlt sich die Verwendung eines Schutzgases im Reaktionsgefäß, z.B. Stickstoff oder Argon. Die Abspaltung der Trialkylsilyl-Schutzgruppen kann z.B. durch Kochen der Zwischenverbindung mit einem Alkohol, wie Methanol oder Ethanol erfolgen.

Die zur Veretherung benötigten Alkohole werden in Form ihrer Alkalisalze zur Umsetzung gebracht, vorzugsweise als Li-, Na-oder K-Salze. Als Lösungs-bzw. Verteilungsmittel wird im allgemeinen der eingesetzte Alkohol im Überschuß verwendet. Es können jedoch auch andere Alkohole wie Propanole, Butanole Alkylenglykole oder ihre Mono-und Di-alkylether verwendet werden. Die Reaktionstemperaturen liegen im allgemeinen zwischen ca. 25 und 130°C, vorzugsweise zwischen ca. 60 und 100°C. Die Reaktionszeiten liegen je nach Temperatur und Reaktionsteilnehmer meistens zwischen 4 und 24 Stunden.

Die Abspaltung der Benzyl-Schutzgruppen $R^2$ erfolgt beispielsweise durch katalytische Hydrierung mittels Palladium auf Kohle und Wasserstoff-Gas in Gegenwart von Spuren Salzsäure oder Aluminium-III-chlorid oder mittels Palladium-hydroxyd und Cyclohexen oder Cyclohexadien-1,3 bzw. -1,4 als Wasserstoff-Generator. Die Hydrierungen werden in Alkoholen als Lösungsmittel durchgeführt.

Die Verseifung der Acyl-Verbindungen mit $R^2 =$ CO-$R^3$ zu Verbindungen mit $R^2 =$ Wasserstoff, erfolgt durch Einwirkung von Basen, wie Ammoniak, Alkylaminen, wie Methyl-oder Ethylamin, den verschiedenen Propyl-oder Butylaminen, oder Alkalihydroxyden oder -carbonaten, wie Natriumhydroxid, Kaliumhydroxid, Natriumcarbonat, Kaliumcarbonat in wäßriger oder alkoholischer (Methanol, Ethanol) Lösung, im allgemeinen bei Temperaturen von ca. 0 bis 100°C, vorzugsweise bei ca. 20 bis 80°C.

Die meisten Verbindungen 1 fallen nach Verfahren A bis C als Gemisch von Purin-7-und -9-Isomeren in wechselnden Mengenverhältnissen an, die gegebenenfalls durch fraktionierte Kristallisation getrennt werden können. Im allgemeinen wird die Isomerentrennung mittels Säulenchromatographie z.B. über Kieselgel durchgeführt. Die Charakterisierung der Isomeren und ihre Prozentgehaltbestimmung erfolgt z.B. durch Hochdruckflüssigkeitschromatographie (HPLC) unter Verwendung von Li-Chrosorb Si 60/5 µm der Firma Merck, Darmstadt, Deutschland.

Die erfindungsgemäßen Verbindungen der Formel 1 besitzen wertvolle pharmazeutische Eigenschaften. Sie sind in vitro und in vivo gegen Verschiedene Viren wirksam und eignen sich daher zur Bekämpfung verschiedener, durch DNS-Viren verursachter Krankheiten, wie Herpes, z.B. Typus simplex 1 und 2, Cytomegalie und RNS-Viren, z.B. Retroviren, sowie Autoimmunkrankheiten und zur Behandlung von Krebs. Arzneimittel, die sie enthalten, können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden solche von mindestens 0,1, vorzugsweise mindestens 0,2 bis höchstens 10, vorzugsweise 8 mg/kg Körpergewicht, bezogen auf das Körpergewicht von 75 kg eines Erwachsenen, verabreicht. Sie werden zweckmäßig in Dosierungseinheiten verabreicht, die mindestens die wirksame Tagesmenge der erfindungsgemäßen Verbindungen, z.B. 30 bis 300, vorzugsweise 50 bis 250 mg enthalten.

Die erfindungsgemäßen Verbindungen können auch in Kombination mit anderen Antivirusmitteln und Immunstimulantien, wie Interferonen verabreicht werden.

Beispiele

1a). 2-Amino-6-isopropoxy-9-(1,3-dihydroxy-2-propoxymethyl)-purin (Verfahren A)

3,70 g (0,076 Mol) 2-Amino-6-isopropoxy-9-(1,3-dibenzyloxy-2-propoxymethyl)-purin werden in einer Mischung von 40 ml absolutem Ethanol und 90 ml Cyclohexen gelöst, 0,50 g Palladiumhydroxyd auf Kohle (20 %ig) zugesetzt und die Reduktionsmischung unter Stickstoff-Schutzgas 72 Stunden (3 Tage) unter Rückfluß erhitzt. Innerhalb dieses Zeitraums werden etwa alle 6 Stunden weitere Portionen von etwa 0,3 g Palladium-Hydroxyd auf Kohle zugegeben. Danach wird der Katalysator abgesaugt, die Reduktionslösung eingedampft und der ölige Rückstand über Grace-Kieselgel mit dem Laufmittel Methylenchlorid/Methanol 20 : 1 auf einer Säule chromatographiert. Nach dem Eindampfen der Hauptfraktion erhält man ein Öl, welches durch Anrühren mit Ether zur Kristallisation gebracht wird. Es wird abgesaugt, mit Ether gewaschen und im Exsikkator getrocknet.
Ausbeute: 1,8 g = 84 % d. Th., weiße Kristalle, Fp. 126°C.
M 297 (massenspektrographisch)
$^1$H-NMR δ-Werte in Dimethylsulfoxid ($Me_2SO$-$d_6$, 270 MHz) : 6,4 (s, 2H - $NH_2$, C-2), 7,95 (s,1H - CH, C-8), 5,5 (s,2H - $N\underline{CH_2}O$), 3,55 (m,1H - $O\underline{CH}(CH_2)_2$), 3,3/3,4 (m,4H - $CH(\underline{CH_2})_2$), 5,45 (m,1H - $O\underline{CH}(CH_3)_2$), 1,35 (d,6H - $OCH(\underline{CH_3})_2$), 4,6 (t,2H -$CH(\underline{CH_2OH})_2$)
Verbindung nach HPLC-Analyse : $\underline{99}$ %ig.

1b). 2-Amino-6-isopropoxy-9-(1,3-diacetoxy-2-propoxymethyl)-purin

kann erhalten werden durch Umsetzung von 2-Amino-6-isopropoxy-(1,3-dihydroxy-2-propoxymethyl)-purin (nach Beispiel 1a)) in überschüssigem Essigsäureanhydrid in Gegenwart von katalytischen Mengen von 4-N-Dimethylamin-pyridin durch 4-tägiges Rühren bei Zimmertemperatur. Eindampfen im Vakuum, Anrühren mit Ether. Weiße Kristalle vom Fp. 142°C.

1c). 2-Amino-6-isopropoxy-9-(1,3-dibenzyloxy-2-propoxymethyl)-purin (Vorstufe)

4,8 g (0,025 Mol) 2-Amino-6-isopropoxy-purin werden in 100 ml Hexamethyldisilazan gelöst, 2,75 g Ammoniumsulfat (wasserfrei) zugegeben und die Reaktionsmischung unter Stickstoff 4 Stunden unter Rückfluß erhitzt. Das überschüssige Hexamethyldisilazan wird im Vakuum unter Stickstoff abdetilliert und der ölige Rückstand in 50 ml Toluol gelöst.

Es werden 7,5 ml Triethylamin zugegeben und unter Stickstoff, Rühren und Eiskühlung 12,8 g (0,04 Mol) 1,3-Dibenzyloxy-2-chlormethoxy-propan zugetropft. Sodann wird 12 Stunden unter Rühren und Stickstoff auf 80°C erhitzt. Das Lösungsmittel Toluol wird im Vakuum abdestilliert, der ölige Rückstand mit 150 ml Methanol versetzt und 30 Minuten unter Rückfluß erhitzt. Nach Abkühlen wird von einer kleinen Menge Nebenprodukt abgesaugt, eingedampft, der Rückstand in Methylenchlorid gelöst, mit Wasser ausgeschüttelt, die organische Phase abgetrennt, über Natriumsulfat getrocknet, eingedampft und der ölige Rückstand über Grace-Kieselgel mit dem Laufmittel Essigester/Cyclohexan 10 : 1 auf einer Säule chromatographiert. Nach dem Eindampfen der Hauptfraktion erhält man ein Öl, welches durch Anrühren mit Ether/Cyclohexan kristallisiert. Es wird abgesaugt, mit Ether gewaschen und im Exsikkator getrocknet.
Ausbeute: 6,0 g = 50 % d. Th., weiße Kristalle, Fp. 93°C.
M 477 (massenspektrographisch)
$^1$H-NMR δ-Werte in Dimethylsulfoxid ($Me_2SO$-$d_6$, 270 MHz) : 6,35 (s,2H - $NH_2$, C-2), 7,95 (s,1H - CH, C-8), 5,5 (s,2H - $N\underline{CH_2}O$), 4,1 (m,1H - $O\underline{CH}(CH_2)_2$), 3,4/3,5 (m,4H - $CH(\underline{CH_2})_2$), 5,5 (m,1H - $O\underline{CH}(CH_3)_2$), 1,3 (d,6H - $OCH(\underline{CH_3})_2$), 4,4 (s,4H -$O\underline{CH_2}Ph$), 7,25 Ph
Verbindung nach HPLC-Analyse : 95,6 %ig.

In analoger Weise lassen sich auch alle anderen beanspruchten Verbindungen mit $R^1$ Alkyl mit 1 bis 6 Kohlenstoffatomen und $R^2$ Wasserstoff oder Benzyl darstellen, z.B. 2-Amino-6-methoxy-9-(1,3-dihydroxy-2-propoxymethyl)-purin, 2-Amino-6-äthoxy-9-(1,3-dihydroxy-2-propoxymethyl)-purin, 2-Amino-6-n-propoxy-9-(1,3-dihydroxy-2-propoxymethyl)-purin, 2-Amino-6-iso-butoxy-9-(1,3-dihydroxy-2-propoxymethyl)-purin, 2-Amino-6-sek-butoxy-9-(1,3-dihydroxy-2-propoxymethyl)-purin.

2. 2-Amino-6-isopropoxy-9-(1,3-dihydroxy-2-propoxymethyl)-purin

Die Verbindung nach Beispiel 1a) kann auch nach Verfahren B wie folgt dargestellt werden:
27,7 g (0,1 Mol) 2-Acetamino-6-isopropoxy-9-acetyl-purin werden zusammen mit 37,8 g (0,1 Mol + 10 % Überschuß) 1,3-Dibenzyloxy-2-acetoxymethoxy-propan und 0,2 g Toluol-4-sulfonsäure in 150 ml wasserfreiem Sulfolan 60 Stunden unter Rühren auf 95°C erhitzt. Die erkaltete Reaktionsmischung wird mit 1500 ml Toluol verdünnt, filtriert und über Grace-Kieselgel chromatographiert. Die Elution erfolgt mit einer Mischung von Methylenchlorid/Methanol (Volumenverhältnis 10 : 1). Das Eluat wird eingedampft, der Rückstand wird in einer Mischung aus 500 ml Methanol und 500 ml flüssigem Ammoniak gelöst und 12 Stunden bei Zimmertemperatur gerührt. Nach Zusatz von etwas Aktivkohle wird abgesaugt, eingedampft und der Rückstand durch Anrühren mit einer Mischung von Ether/Cyclohexan zur Kristallisation gebracht.
Ausbeute: 19,5 g = 40 % d.Th., weiße Kristalle vom Fp. 90°C.
Die Substanz ist 2-Amino-6-isopropoxy-9-(1,3-dibenzyloxy-2-propoxymethyl)-purin, identisch mit Verbindung nach Beispiel 1b). Sie kann wie unter Beispiel 1a) beschrieben, in die Titelverbindung überführt werden.

Ausgangsstoffe:

2-Acetamino-6-isopropoxy-9-acetyl-purin wurde durch Acetylierung von 2-Amino-6-isopropoxy-purin mit Essigsäureanhydrid gewonnen.

1,3-Dibenzyloxy-2-acetoxymethoxy-propan wurde durch Umsetzung von 1,3-Dibenzyloxy-2-chlormethoxy-propan mit Kaliumacetate in Aceton bei Zimmertemperatur gewonnen.

3. 2-Amino-6-isopropoxy-9-(1,3-dihydroxy-2-propoxymethyl)-purin

Verbindung nach Beispiel 1a) kann auch nach Verfahren C wie folgt dargestellt werden:
Eine Mischung von 17,0 g (0,1 Mol) 2-Amino-6-chlor-purin in 250 ml Hexamethyldisilazan und 3,3 g (0,025 Mol) Ammoniumsulfat (wasserfrei) wird 3 Stunden unter Stickstoff-Schutzgas unter Rühren zum Rückfluß erhitzt. Danach wird das überschüssige Hexamethyldisilazan im Vakuum abdestilliert, der Rückstand in 300 ml Toluol gelöst, etwas Quecksilber-II-cyanid zugesetzt und bei 80°C unter Stickstoff und Rühren eine Lösung von 35,3 g (0,1 Mol + 10 % Überschuß) 1,3-Dibenzyloxy-2-chlormethoxy-propan in 100 ml Toluol zugetropft. Ist alles eingetragen, wird noch 3 Stunden bei 80°C nachgerührt. Danach wird das Lösungsmittel Toluol im Vakuum abdestilliert, der Rückstand mit 400 ml Methanol versetzt und 30 Minuten unter Rückfluß erhitzt. Es wird eingedampft, der Rückstand in Methylenchlorid gelöst und über eine Grace-Kieselgel-Säule chromatographiert. Es wird mit einer Mischung von Essigester/Cyclohexan (Volumenverhältnis 1 : 1) eluiert. Die Hauptfraktion wird eingedampft und der Rückstand (2-Amino-6-chlor-9-(1,3-dibenzyloxy-2-propoxymethyl)-purin, Rohprodukt) direkt mit überschüssigem Natrium-isopropylat in Isopropanol (1 Stunde Rückfluß) umgesetzt. Nach Abkühlung wird mit verdünnter Salzsäure neutralisiert,

von Salzen abgesaugt und bis auf 1/4 des Volumens eingedampft. Die Lösung wird mit 100 ml Cyclohexan und 0,5 g Palladiumhydroxyd auf Kohle versetzt und dann werden, wie unter Beispiel 1a) beschrieben, die Benzylschutzgruppen abhydriert. Nach analoger Aufarbeitung erhält man die identische Verbindung 1a) mit einem Fp. 124°C.

## Ansprüche

1. Verbindungen der Formel 1

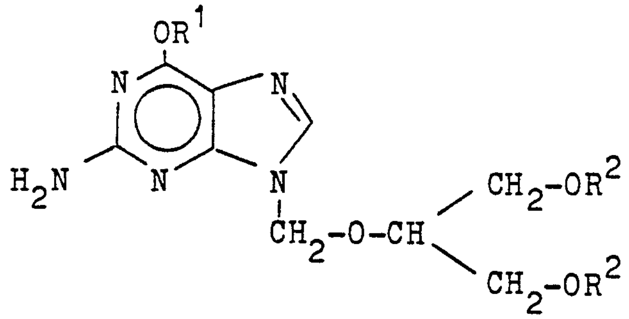

1

worin
$R^1$ eine Alkylgruppe mit 1 bis 6 Kohlenstoffatomen,
$R^2$ Wasserstoff, eine Benzylgruppe oder eine Acylgruppe CO-$R^3$ ist, wobei
$R^3$ Alkyl mit 1 bis 6 Kohlenstoffatomen, Benzyl oder Phenyl ist.

2. Verbindung nach Anspruch 1, dadurch gekennzeichnet, daß $R^1$ Isopropyl und $R^2$ Wasserstoff und/oder Acetyl bedeuten.

3. Verfahren zur Herstellung von Verbindungen der Formel 1 gemäß Anspruch 1, dadurch gekennzeichnet, daß man

A) Verbindungen der Formel 2

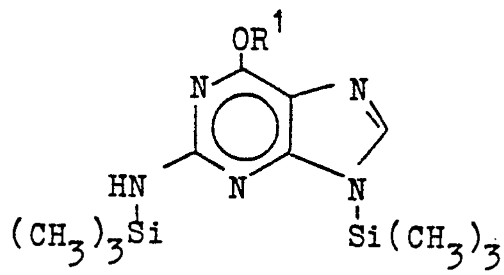

2

in denen $R^1$ wie oben definiert ist,
mit Halogen-, insbesondere Chlorverbindungen der Formel 3 umsetzt,

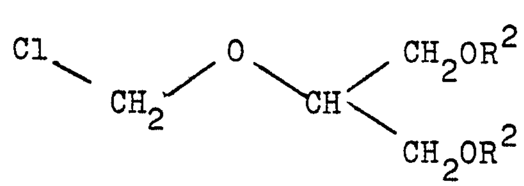

3

in denen $R^2$ wie oben definiert ist,
und daß man anschließend die Trimethylsilyl-bzw. Benzyl-Schutzgruppe abspaltet und den Acylrest gegebenenfalls verseift;

B) Verbindungen der Formel 4

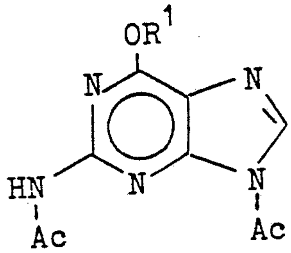

4

in denen $R^1$ wie oben definiert ist, mit reaktiven Acetoxy-Verbindungen der Formel 5 umsetzt,

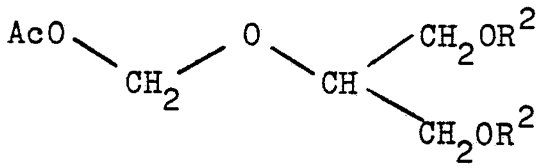

5

in der $R^2$ Benzyl oder Acyl und $R^3$ wie oben definiert ist, und daß man die Benzyl-Schutzgruppen abspaltet und die Acylreste verseift;

C) 2-Amino-6-chlorpurin der Formel 6, gegebenenfalls als Bis-(trimethylsilyl)-Verbindung der Formel 7

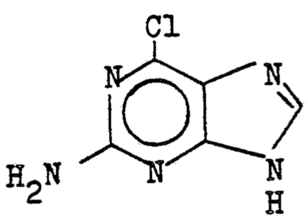

6

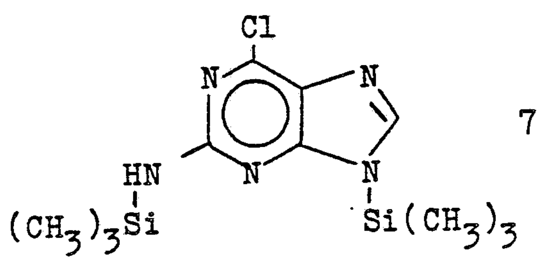

7

mit Halogen-, insbesondere Chlorverbindungen der Formel 3 umsetzt,

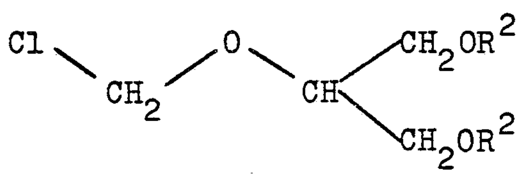

3

in denen $R^2$ wie oben definiert ist, und die Umsetzungsprodukte der Formel 8 oder 9

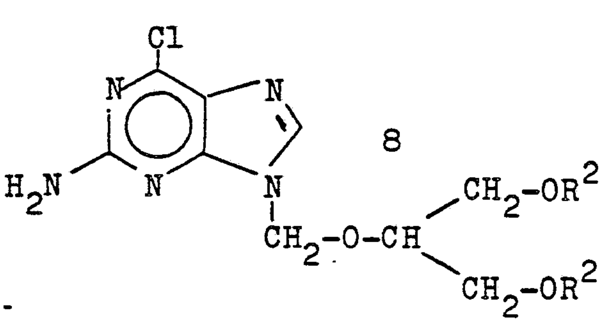

8

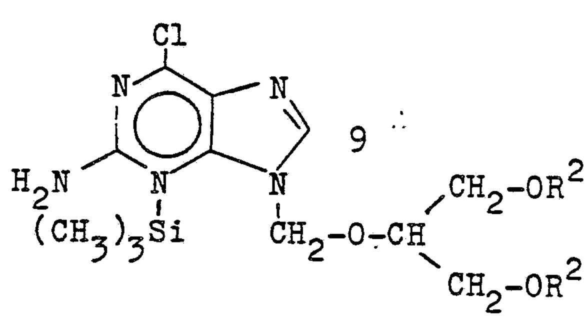

9

nach Abspaltung der Trimethylsilyl-Schutzgruppe in Verbindung 9 mit Alkali-Alkoholaten der Formel 10

Akalimetall-$OR^1$ 10

wobei $R^1$ wie oben definiert ist und Alkalimetall Li, Na oder K bedeutet, verethert und die Benzyl-

Schutzgruppen $R^2$ abspaltet.

4. Arzneimittel, gekennzeichnet durch einen Gehalt an einer wirksamen Menge wenigstens einer Verbindung der Formel 1 gemäß Anspruch 1.

Patentansprüche fur die folgenden Vertragsstaaten: Au, Gr

1. Verfahren zur Herstellung von Verbindungen der Formel 1,

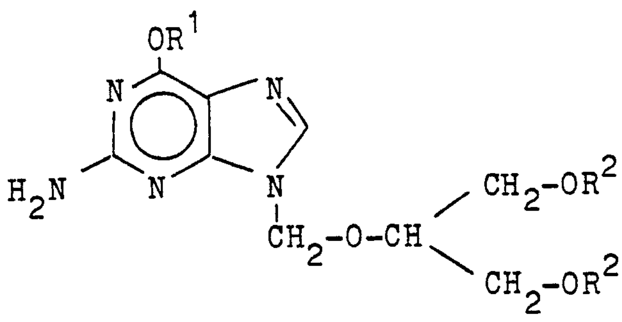

1

worin
$R^1$ eine Alkylgruppe mit 1 bis 6 Kohlenstoffatomen,
$R^2$ Wasserstoff, eine Benzylgruppe oder eine Acylgruppe CO-$R^3$ ist, wobei
$R^3$ Alkyl mit 1 bis 6 Kohlenstoffatomen, Benzyl oder Phenyl ist,
dadurch gekennzeichnet, daß man

A) Verbindungen der Formel 2

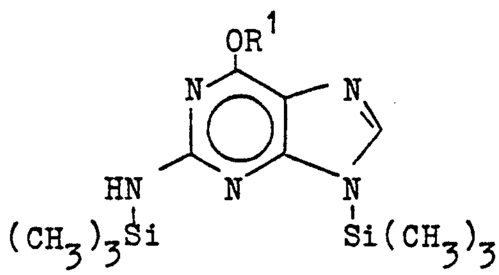

2

in denen $R^1$ wie oben definiert ist,
mit Halogen-, insbesondere Chlorverbindungen der Formel 3 umsetzt,

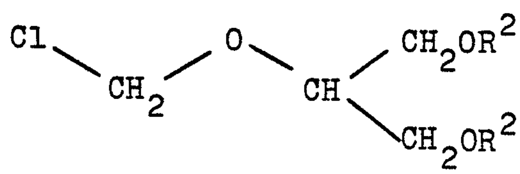

3

in denen $R^2$ wie oben definiert ist,
und daß man anschließend die Trimethylsilyl-bzw. Benzyl-Schutzgruppe abspaltet und den Acylrest gegebenenfalls verseift; oder

B) Verbindungen der Formel 4

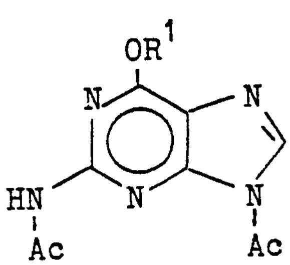

4

in denen $R^1$ wie oben definiert ist, mit reaktiven Acetoxy-Verbindungen der Formel 5 umsetzt,

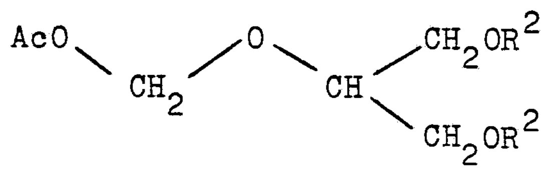

5

in der $R^2$ Benzyl oder Acyl und $R^3$ wie oben definiert ist,
und daß man die Benzyl-Schutzgruppen abspaltet und die Acylrest verseift; oder

C) 2-Amino-6-chlorpurin der Formel 6, gegebenenfalls als Bis-(trimethylsilyl)-Verbindung der Formel 7

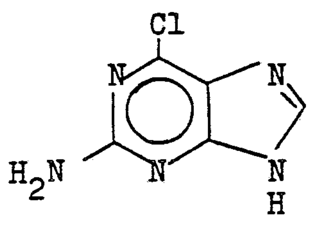

6

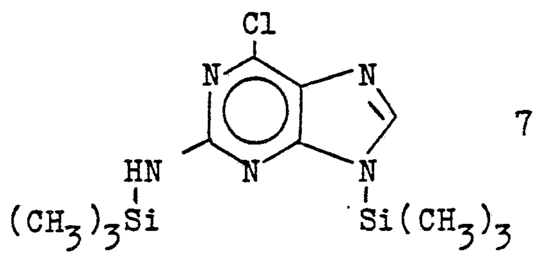

7

mit Halogen-, insbesondere Chlorverbindungen der Formel 3 umsetzt,

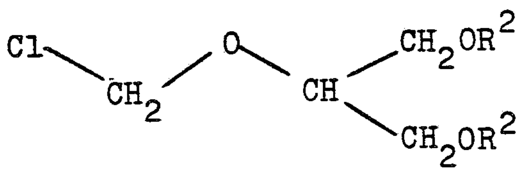

3

in denen $R^2$ wie oben definiert ist,
und die Umsetzungsprodukte der Formel 8 oder 9

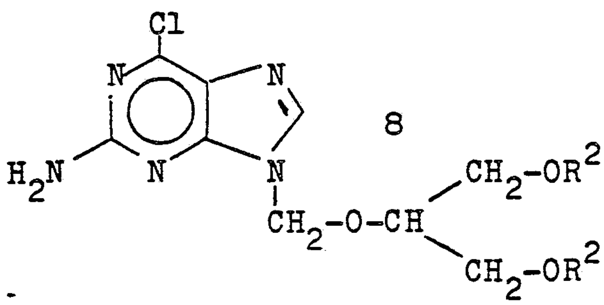

8

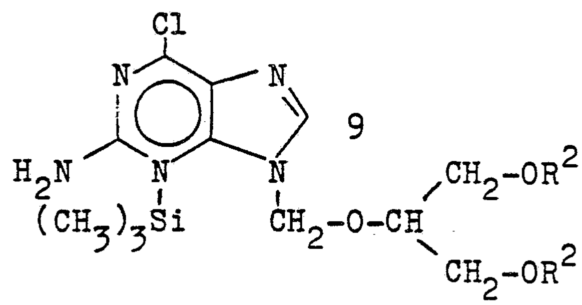

9

nach Abspaltung der Trimethylsilyl-Schutzgruppe in Verbindung 9 mit Alkali-Akoholaten der Formel 10

Alkalimetall-$OR^1$ 10

wobei $R^1$ wie oben definiert ist und Alkalimetall Li, Na oder K bedeutet, verethert und die Benzyl-Schutzgruppen $R^2$ abspaltet.

2. Verfahren nach Anspruch 1, dadurch gekennzeichnet, daß man Verbindungen der Formel 1 mit $R^1$ = Isopropyl und $R^2$ = H und/oder Acetyl herstellt.

3. Verfahren zur Herstellung eines Arzneimittels, dadurch gekennzeichnet, daß man eine wirksame Menge wenigstens einer Verbindung der Formel 1 wie erhalten nach den Verfahren gemäß Anspruch 1 oder 2 mit einem physiologisch annehmbaren Träger und ggf. weiteren Zusatz-und/oder Hilfsstoffen in eine geeignete Darreichungsform bringt.